Europäisches Patentamt

European Patent Office

Office européen des brevets

(19)

(11) Veröffentlichungsnummer : **0 168 559**
**B1**

(12) **EUROPÄISCHE PATENTSCHRIFT**

(45) Veröffentlichungstag der Patentschrift :
28.09.88

(51) Int. Cl.⁴ : **A 61 B 17/22**

(21) Anmeldenummer : 85104659.9

(22) Anmeldetag : 17.04.85

(54) Vorrichtung zum Orten und Positionieren von Konkrementen.

(30) Priorität : 18.07.84 DE 3426398

(43) Veröffentlichungstag der Anmeldung :
22.01.86 Patentblatt 86/04

(45) Bekanntmachung des Hinweises auf die Patenterteilung : 28.09.88 Patentblatt 88/39

(84) Benannte Vertragsstaaten :
BE FR GB IT NL SE

(56) Entgegenhaltungen :
DE-A- 3 122 056
DE-A- 3 220 751
DE-B- 2 351 247
DE-B- 2 650 212
DE-B- 2 722 252
PROSPEKT DORNIER LITHOTRIPTER ZMV 207-
84035000

(73) Patentinhaber : DORNIER SYSTEM GmbH
Postfach 1360
D-7990 Friedrichshafen (DE)

(72) Erfinder : Forssmann, Bernd, Dr.
Salemweg 6
D-7990 Friedrichshafen 1 (DE)
Erfinder : Gerth, Hans-Heinrich, Dr.
Hans-Dieter-Strasse 26
D-7758 Meersburg (DE)
Erfinder : Wess, Othmar, Dr.
Hersbergweg 6
S-7997 Immenstaad (DE)

(74) Vertreter : Landsmann, Ralf, Dipl.-Ing.
Kleeweg 3
D-7990 Friedrichshafen 1 (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

EP 0 168 559 B1

**Beschreibung**

Die Erfindung betrifft eine Vorrichtung zum Orten und Positionieren von Konkrementen nach dem Oberbegriff des Anspruchs 1.

Aus der DE-PS 23 51 247 ist eine Einrichtung zum Zerkleinern von im Körper eines Lebewesens befindlichen Kronkrementen mit einer Fokussierungskammer, die Teil eines Rotationsellipsoids ist und in deren ein Brennpunkt Stoßwellen durch Funkenentladung erzeugbar sind, bekannt. Damit ist eine Zerkleinerung von z. B. Nierensteinen ohne operativen Eingriff oder Einführung von Sonden möglich. Der Nierenstein wird beispielsweise mittels zweier Röntgenabbildungssysteme in seiner Größe und seiner Lage im Körper des Patienten lokalisiert. Zur Vermeidung hoher Strahlenbelastungen werden nur sowenig Röntgenaufnahmen wie notwendig durchgeführt. Dann wird der Stein von fokussierten Stoßwellen zu einem feinen Gries zerkleinert, der auf natürliche Weise aus dem Körper herausgeschwemmt wird.

Aus der DE-PS 27 22 252 ist eine Einrichtung zur räumlichen Ortung von Konkrementen mit Ultraschall bekannt. Voraussetzung für ein zufriedenstellendes Arbeiten ist, daß der Stein und die ihn umgebenden Organe in einer festen Ruhelage sind. Durch die natürliche Eigenbewegung des Steines und der Organe infolge der Atmung pendelt der Stein immer wieder durch die Ortungsebene. Aus dem sich ständig wechselnden Ultraschallbild kann der Stein nur für kurze Zeit, wenn er durch die Ortungsebene schwingt, identifiziert werden. Bei der Einjustierung des Steines in den Brennpunkt, der in der Ortungsebene liegt, kann durch die Überlagerung der natürlichen Eigenbewegung mit der Positionierbewegung die Information über die Steinlage ganz verloren gehen, so daß der Ortungsvorgang zeitraubend wiederholt werden muß.

Zeitraubend ist der Vorgang insbesondere dann, wenn die Positionierung des Konkrements (z. B. durch Verschieben des Patienten gegen den Reflektor) unabhängig von den Ortungseinrichtungen erfolgt.

Aufgabe der Erfindung ist es, für die berührungsfreie Zerkleinerung von Konkrementen durch Stoßwellen eine Vorrichtung zum räumlichen Orten der Konkremente und zum Positionieren der Konkremente zu schaffen, mit der die Konkremente sicher erkannt werden und die Konkremente einfach, schnell und zuverlässig in den Brennpunkt eines Reflektors positioniert und dort gehalten werden können.

Diese Aufgabe wird erfindungsgemäß gelöst durch Vorrichtungen mit den in den Ansprüchen genannten Merkmalen.

Die Korrelation der Ortungssysteme ermöglicht bei Verwendung zweier Bildschirme ein schnelleres Auffinden des Konkrements, eine höhere Erkennungssicherheit und eine einfache Beobachtung der Atemexkursion des Konkrements durch das Bedienungspersonal. Die Korrelation der Ortungssysteme mit der Positionierung ermöglicht ein einfaches, schnelles Nachführen des Konkrements in den Fokusbereich entweder durch das Bedienungspersonal oder selbsttätig durch automatische Bildverarbeitung z. B. durch interaktive Bildschirmsysteme. Die Verwendung mindestens eines Ultraschall-Ortungssystems ermöglicht es, die Bewegungen des Konkrements in Echtzeit und ohne Strahlenbelastung zu überwachen. Die Stoßwellen können immer ausgelöst werden, wenn der Stein bei seiner durch die Atembewegung bedingten Pendelbewegung den Fokus passiert. Dies erhöht die Effizienz der Zerkleinerung, schont den Patienten durch kürzere Behandlungszeit und verlängert die Lebensdauer der Funkenstrecke.

Das erfindungsgemäße kombinierte Ultraschall-Röntgen-Ortungssystem vereinigt die Vorteile von Ultraschall — Unschädlichkeit — und Röntgen — hohe Auflösung — in idealer Weise, sowohl im Hinblick auf die Nierenstein-, als auch auf die Gallensteinzertrümmerung. Bei der Nierensteinzerkleinerung tritt der Vorteil des Röntgens bei der Beurteilung des Behandlungsablaufes und Behandlungserfolges zu tage, während der Ultraschall nur zur Lagebestimmung des Konkrements verwendet wird. Bei der Gallensteinzertrümmerung wird das Röntgen vornehmlich zur Vororientierung und zur Auffindung der Steinlage eingesetzt, um die optimale Schnittebene zu finden, so daß die Vorteile des Ultraschalls voll ausgeschöpft werden können. Möglich ist z. B. die Verwendung eines Röntgenortungssystems als Orientierungshilfe zu einem Ultraschall-Ortungssystem (B-Scan). Da ein einzelnes Röntgensystem im allgemeinen nur zwei der drei Raumkoordinaten des Zielobjekts erfassen kann, schließen die Röntgenbildebene und die Ultraschallbildebene bevorzugt einen Winkel von 90° ein, damit die fehlende dritte Koordinate aus dem Ultraschallbild entnommen werden kann. Beide Systeme sind darüber hinaus so angeordnet, daß sie den Fokuspunkt $F_2$ des Ellipsoids abbilden. In Fällen mit nicht ausreichender Erkennungssicherheit dient die Röntgenortung zur Kontrolle von zwei der drei Koordinaten (erhöht somit die Erkennungssicherheit beträchtlich), oder sie wird primär eingesetzt zur Vorjustierung des Konkrements in zwei Dimensionen und ermöglicht damit eine vereinfachte und schnellere Erkennung und Positionierung in der dritten Koordinate durch das Ultraschallortungssystem.

Etwaige periodische Bewegungen des Konkrements (hauptsächlich durch die Atembewegung) können in Echtzeit im Ultraschallbild beobachtet werden. Zur besseren Kontrolle derartiger periodischer Bewegungen läßt sich das Ultraschall-Ortungssystem um eine Achse drehen, die durch das Zentrum des Ultraschallschwingers und den Fokus $F_2$ gegeben ist (Achse $U_0$). Sobald der Positioniervorgang in der beschriebenen Weise durchgeführt ist, läßt sich durch eine einfache axiale Drehung des Ultraschallschwingers unter Beobachtung des Ultraschallbildes die Ultraschallbildebene parallel zu der Hauptschwingungsrichtung des periodisch bewegten Ziels ausrichten. Damit ist ohne komplizierte variable Koordinatentransformation eine zuverlässige Zielkontrolle in Echtzeit möglich.

Die in einer Ausführungsform der Erfindung vorgesehenen Verdrehmöglichkeiten des Ultraschallsystems um zwei Achsen ermöglichen also solche Einstellungen, daß die Pendelbewegungen des Steins stets in der Ultraschall-Schnittebene liegen, so daß der Stein ständig am Ultraschall-Bildschirm zu beobachten ist. Ein Vorteil dieser Verdrehmöglichkeiten ist, daß der Stein von verschiednen Seiten dargestellt werden kann und damit das Volumen über einen On-Line-Rechner berechnet werden kann. Daraus ergeben sich neue Informationen für die Dosierung der Stoßwellenenergie.

Bei einer Ausführungsform der Erfindung ist zusätzlich auch die Positioniervorrichtung mit den beiden Ortungssystemen korreliert. Dies ist z. B. dadurch erreicht, daß zwei Koordinatenrichtungen der Positionierungen mit der Ultraschall-Bildebene zusammenfallen und die dritte annähernd senkrecht dazu steht. Durch Verfahren des Patienten in Richtung der dritten Koordinate taucht das Ziel im Bildfeld auf und kann mit Hilfe der im Ultraschall-Bild dargestellten beiden anderen Koordinatenrichtungen leicht in den markierten Fokuspunkt justiert werden. Da nur noch Korrekturen in Richtung der in dem Ultraschall-Bild dargestellten Koordinaten erfolgen, bleibt das Ziel kontinuierlich sichtbar und kann nicht unbeabsichtigt aus dem Bildfeld verschwinden. Der Ortungsvorgang kann dadurch wesentlich schneller abgewickelt werden, da auch größere Korrekturschritte (eventuell über Daumenhebelsteuerung) kontrollierbar durchführbar sind. Die Verschiebung in der Ultraschall-Schnittebene kann auch mit Hilfe eines Rechners automatisiert erfolgen. Die Auswertung kann beispielsweise auf einem interaktiven Bildschirm erfolgen, z. B. mit einem Lichtgriffel, den der Arzt auf dem Bildschirm auf den Steinort legt oder durch Eingabe des Steinorts in die Tastatur eines Rechners.

Bei dieser Ausführungsform der Erfindung ist somit zuverlässig verhindert, daß — wie es bei unkorrelierten Systemen vorkommen kann — das bereits im Ultraschall-Bild dargestellte Ziel aus der Darstellung verschwindet und ein erneuter zeitaufwendiger Suchprozeß begonnen werden muß. Die Gefahr des unbeabsichtigten Verschwindens des Ziels vom Bildschirm besteht beim jetzigen Stand der Ortungstechnik darin, daß das Ultraschall-Bild nur eine dünne Scheibe aus dem Objektfeld abbildet und eine Darstellung des Ziels erst dann ermöglicht, wenn das Ziel bereits in einer Koordinate richtig justiert ist. Bei verdreht gegeneinander angeordneten Koordinatensystemen für die Patientenpositionierung und die Ultraschall-Bilddarstellung kann jede Positionskorrektur in einer Koordinate der Patientenpositionierung zum Verschwinden des Bildes führen.

Eine erfindungsgemässe Ausführungsform mit zwei Ultraschallsystemen deren Bildebenen senkrecht aufeinander stehen, ermöglicht eine Echtzeitkorrektur des Konkrementbewegungen durch eine automatische Nachführung.

Bei dieser Ausführungsform erscheint das einmal positionierte Konkrement in beiden Ultraschall-Bildern. Je zwei der drei Raumkoordinaten sind in einem Ultraschall-System dargestellt, z. B. xy in System 1 und yz in System 2.

Aus den Echtzeit-Ultraschall-Bildern lassen sich alle notwendigen Korrekturdaten für eine Echtzeit-Positionskorrektur entnehmen, wie Bewegungsrichtung im Raum, Geschwindigkeit und Beschleunigung. Über Servomotoren wird die Konkrementposition korrigiert, so daß das Konkrement zuverlässig im zweiten Fokus gehalten wird.

Die Ermittlung der Korrekturdaten in jeweils zwei Koordinaten (z. B. x, y, System 1) kann auf verschiedene Weise erfolgen, z. B. mit Hilfe von Bildspeichern und Korrelationstechniken, mit Hilfe einer automatischen Zeichenerkennung oder durch Anwendung von je einem Positionsdetektor pro Ultraschall-System.

Die letztgenannte Möglichkeit ist besonders einfach, da derartige Positionsdetektoren kommerziell erhältlich sind und eine Bewegung eines definierten Bildpunktes in zwei Dimensionen nach Richtung, Geschwindigkeit und Beschleunigung erfassen können. In Verbindung mit zwei Ultraschall-Systemen sind damit alle notwendigen Korrekturdaten vorhanden.

Eine Echtzeit-Positionskorrektur ist für die Nierensteinzertrümmerung, wie für die Gallensteinzertrümmerung anwendbar.

Die Erfindung wird anhand der Figuren 1 bis 4 näher erläutert.

Figur 1 zeigt eine Ausführungsform eines erfindungsgemäßen Ortungssystems.

Die Figuren 2 bis 4 zeigen Anzeigen auf dem Röntgen-Bildschirm und auf dem Ultraschall-Bildschirm bei drei beispielhaften Vorgängen zum Orten und Positionieren eines sich durch die Atmung bewegenden Konkrements.

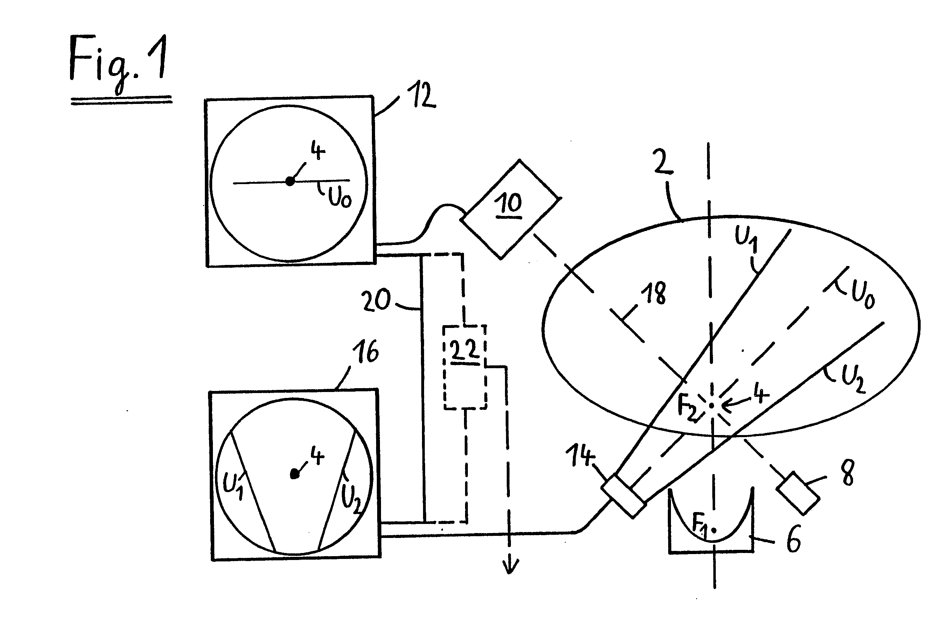

Figur 1 zeigt schematisch den Körper 2 eines Patienten im Querschnitt mit dem zu zerkleinernden Konkrement 4, den Reflektor 6, in dessen ersten Brennpunkt $F_1$ die Stoßwellenquelle liegt und in dessen zweiten Brennpunkt $F_2$ das Konkrement 4 positioniert werden soll. Nicht gezeigt sind die Vorrichtungen zum Verschieben (Positionieren) des Patientenkörper 2 und etwaige Vorrichtungen zum Ankoppeln oder Einleiten der Stoßwellenenergie in den Körper. Das erfindungsgemäße Ortungssystem besteht aus einem Röntgen-Ortungssystem mit Röntgenröhre 8, Bildverstärker 10 und Röntgen-Bildschirm 12 und einem Ultraschall-Ortungssystem, bestehend aus Ultraschall-Kopf 14 und Ultraschall-Bildschirm 16. Die Korrelation der beiden Anzeigen 12, 16 ist hier durch die Verbindungsleitung 20 angedeutet. Gestrichelt gezeichnet ist die alternative Ausführung mit einem Korrelationsrechner 22, dessen Ausgang zu den Stellmotoren der Patientenpositionierung führt. Die Koordinaten werden bei Verwendung des Rechners 22 vom Rechner 22 aus beiden Bildern bestimmt. Bei einer Fehlbestimmung kann automatisch berechnet werden, ob die Koordinaten miteinander verträglich sind, da die zwei Koordinaten aus dem Ultraschallsystem und die zwei Koordinaten aus dem Röntgensystem eine Überbestimmung der drei Raumkoordinaten des Konkrements bilden. Die Reflektorachse $F_1$, $F_2$, der Röntgen-Zentralstrahl 18 und der Mittelstrahl $U_0$ der Ultraschall-Schnittebene $U_1$, $U_2$ schneiden sich im Brennpunkt $F_2$. Diese drei Achsen müssen jedoch nicht in einer gemeinsamen Ebene liegen.

Mit dem Ultraschall-Kopf 14, beispielsweise einem Sektorscan oder Ultraschall-array wird ein zweidimensionales Bild aufgebaut, wie es aus der medizinischen Diagnostik als B-Scan bekannt ist. Das Ultraschall-System erzeugt ein Entfernungsbild (Laufzeitmessung), wobei eine Ortungsebene oder Schnittebene, begrenzt durch die Strahlen $U_1$ und $U_2$ abgetastet wird. Am Ultraschall-Bildschirm 16 ist die abgetastete Ebene $U_1$, $U_2$ in Draufsicht dargestellt, die hier den Stein 4 enthält. Das Ultraschall-System ist hier so eingestellt, daß der Röntgen-Zentralstrahl 18 ganz in der Ultraschall-Ortungsebene $U_1$, $U_2$ liegt. Der Ultraschall-Kopf 14 kann zur Vermeidung von Schädigungen durch die Stoßwellen im Schattenbereich des Reflektor 6 angeordnet sein. Das Röntgen-Abtastsystem erzeugt am Bildschirm 12 ein « normales » zweidimensionales Bild vom Konkrement 4. Auf dem Bildschirm 12 ist die Projektion der Ultraschall-Schnittebene ($U_0$) eingespielt.

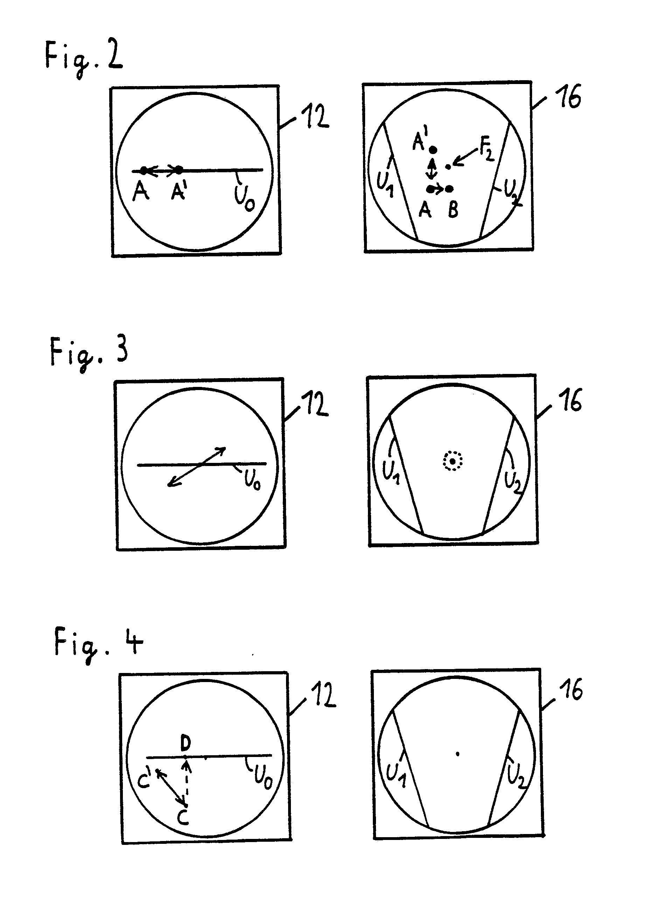

Anhand der Figuren 2, 3 und 4 werden drei Ortungsbeispiele beschrieben.

In Figur 2 ist ein Pendeln des Konkrements durch die Atemexkursion sowohl am Röntgen-Bildschirm 12 als auch am Ultraschall-Bildschirm 16 zu erkennen. Der Stein pendelt dann bereits in der Ultraschall-Ebene von A nach A' und kann durch eine translatorische Bewegung von A nach B so verschoben werden, daß er durch den Fokuspunkt $F_2$ pendelt. Die Verschiebung kann zur Reduktion der Strahlenbelastung am Ultraschall-Bildschirm 16 verfolgt werden.

In Figur 3 ist am Röntgen-Bildschirm 12 ein Pendeln des Steines durch den Fokus zu erkennen. Am Ultraschall-Bildschirm 16 ist lediglich ein periodisches Aufblitzen und Verschwinden des Steinbildes am Fokusort oder auf $U_0$ zu erkennen. Der Ultraschall-Kopf 14 wird nun z. B. um die Achse $U_0$ um den Winkel gedreht, der am Röntgen-Bildschirm 12 zwischen Pendelrichtung und Ultraschall-Ebene auftrat. Der Stein schwingt dann in der Ultraschall-Ebene und ist am Ultraschall-Bildschirm 16 ständig beobachtbar. Das Röntgengerät 8 kann zur Schonung des Patienten ausgeschaltet werden. Eventuell kann eine zweite Translation notwendig sein, um den Stein in den Fokuspunkt zu positionieren.

In Figur 4 ist als allgemeiner Fall der Stein am Ultraschall-Bildschirm noch nicht erkennbar. Am Röntgen-Bildschirm ist zu erkennen, daß der Stein außerhalb der Ultraschall-Ebene von C nach C' schwingt. Der Patient wird nun translatorisch verschoben, bis der Stein durch die am Röntgen-Bildschirm eingespielte Ultraschall-Ebene (Verschiebung C nach D) und durch weitere Verschiebung durch den Fokuspunkt pendelt. Dabei taucht der Stein zeitweise im Ultraschall-Bild auf. Als zweiter Schritt wird die Ultraschall-Ebene entweder um die Achse 18 oder wie bei Figur 3 beschrieben um die Achse $U_0$ gedreht, bis die Pendelbewegung voll in der Ultraschall-Ebene erfolgt. Weiteres Vorgehen wie in den beiden vorher beschriebenen Beispielen. Die hier angegebenen Positionierungsschritte können auch rechnergesteuert durchgeführt werden, wenn der Arzt einem Rechner die Steinlage aus den Bildschirmanzeigen angibt.

**Patentansprüche**

1. Vorrichtung zum räumlichen Orten von Konkrementen (4), die sich in Körpern (2) von Lebewesen befinden und mit Stoßwellen berührungslos zerkleinert werden, und zur Positionierung der Konkremente (4) in den Brennpunkt (F2) eines Stoßwellenreflektors (6) mit zwei Ortungssystemen, dadurch gekennzeichnet, daß das eine Ortungssystem auf Röntgenbasis arbeitet und das andere Ortungssystem auf B-Scan Ultraschallbasis arbeitet und drehbar gebildet ist, und daß die beiden Ortungssysteme miteinander (20) und/oder mit einer Positioniervorrichtung korreliert sind.

2. Vorrichtung nach Anspruch 1, dadurch gekennzeichnet, daß in den Bildschirmen (12, 16) der Ortungssysteme (14, 8, 10) jeweils Bezugspunkte des anderen Systems eingespielt sind.

3. Vorrichtung nach Anspruch 2, dadurch gekennzeichnet, daß der Brennpunkt (F2) in beide Anzeigen (12, 16) eingespielt ist, bei Drehbewegungen automatisch nachgeführt wird und daß die Ultraschallebene ($U_1$, $U_2$) oder der Zentralstrahl ($U_0$) des Ultraschallsystems in den Röntgenbildschirm (12) eingespielt ist.

4. Vorrichtung nach einem der vorhergehenden Ansprüche, dadurch gekennzeichnet, daß der Zentralstrahl (18) des Röntgenortungssystems ganz in der Bildebene ($U_1$, $U_2$) des Ultraschallsystems verläuft, daß also die Röntgenbildebene und die Ultraschallbildebene senkrecht aufeinander stehen.

5. Vorrichtung nach einem der vorhergehenden Ansprüche, dadurch gekennzeichnet, daß für den Ultraschallkopf (14) Drehmöglichkeiten um seine eigene Achse ($U_0$) und/oder um den Zentralstrahl (18) des Röntgenortungssystems und/oder um die Rotationsachse des Stoßwellenreflektors (6) vorhanden sind.

6. Vorrichtung nach Anspruch 1, dadurch gekennzeichnet, daß mindestens ein Ultraschall-Ortungssystem vorhanden ist und daß zwei Achsen einer Positioniervorrichtung mit der Ultraschallbildebene zusammenfallen und über ein Rechnersystem die Koordinaten des Steins am Ultraschall-bildschirm ausgewertet werden und über eine Regelvorrichtung der Nierenstein so bewegt wird, daß er in den zweiten Brennpunkt (F2) gelangt.

7. Vorrichtung nach einem der vorhergehenden Ansprüche, dadurch gekennzeichnet, daß mindestens ein Ultraschallortungssystem und ein Röntgenortungssystem vorhanden sind, und daß zwei Achsen der Positioniervorrichtung in der Ultraschallschnittebene verlaufen und die dritte Achse der Positioniervorrichtung längs des Zentralstrahls (18) des Röntgenortungssystems verläuft, wobei über eine Recheneinheit die Koordinaten des Nierensteins bestimmt werden und über eine Steuerung eine translatorische Bewegung des Nierensteins in den zweiten Brennpunkt (F2) erfolgt.

8. Vorrichtung nach Anspruch 1, dadurch gekennzeichnet, daß zwei Ultraschall-Ortungssysteme verwendet werden, deren Bildebenen senkrecht aufeinander stehen.

9. Vorrichtung nach einem der vorgenannten Ansprüche, dadurch gekennzeichnet, daß die Überwachung des Steinortes (4) durch eine automatische Hell/Dunkel-Abtastung erfolgt.

10. Vorrichtung nach einem der vorhergehenden Ansprüche, dadurch gekennzeichnet, daß das Konkrement (4) mit Hilfe von Bildverarbeitung und Servomotoren selbstätig in den Brennpunkt ($F_2$) nachgeführt wird.

## Claims

1. Apparatus for the location in space of concrements (4) which are situated in the bodies (2) of living organisms and are broken down without contact by means of shock waves, and for positioning the concrements (4) in the focus (F2) of a shock wave reflector (6) by means of two locating systems, characterised in that one locating system operates of the basis of X-rays and the other locating system operates on the basis of B-Scan ultrasound and is rotatably constructed and in that the two locating systems are correlated with one another (20) and/or with a positioning device.

2. Apparatus according to claim 1, characterised in that the image screen (12, 16) of each locating system (14, 8, 10) displays reference points of the other system.

3. Apparatus according to claim 2, characterised in that the focus (F2) is displayed in both screens (12, 16) and is automatically moved to stay in the screen when movements of rotation take place and in that the ultrasound plane ($U_1$, $U_2$) or the central beam ($U_0$) of the ultrasound system is displayed on the X-ray screen (12).

4. Apparatus according to one of the preceding claims, characterised in that the central beam (18) of the X-ray locating system runs entirely in the image plane ($U_1$, $U_2$) of the ultrasound system so that the plane of the X-ray image and the plane of the ultrasound image are perpendicular to one another.

5. Apparatus according to one of the preceding claims, characterised in that the ultrasound head (14) is provided with possibilities of rotating about its own axis ($U_0$) and/or about the central beam (18) of the X-ray locating system and/or about the axis of rotation of the shockwave reflector (6).

6. Apparatus according to claim 1, characterised in that at least one ultrasound locating system is provided and in that two axes of a positioning device coincide with the plane of the ultrasound image plane and the coordinates of the stone appearing on the screen for the ultrasound image are worked out by a computer system and the kidney stone is displaced by a control device so that it moves into the second focus (F2).

7. Apparatus according to one of the preceding claims, characterised in that at least one ultrasound locating system and one X-ray locating system are provided and in that two axes of the positioning device run in the ultrasound sectional plane and the third axis of the positioning device extends along the central beam (18) of the X-ray locating system, the coordinates of the kidney stone being determined by a computer unit and a movement of translation of the kidney stone into the second focus (F2) being carried out by means of a control device.

8. Apparatus according to claim 1, characterised in that two ultrasound locating systems having their image plane perpendicular to one another are employed.

9. Apparatus according to one of the preceding claims, characterised in that monitoring of the locality (4) of the stone is effected by automatic light/dark scanning.

10. Apparatus according to one of the preceding claims, characterised in that the concrement (4) is automatically moved on into the focus (F2) by means of image processing and servo motors.

## Revendications

1. Dispositif pour détecter dans l'espace des concrétions (4) qui se trouvent dans le corps (2) d'un être vivant et qui doivent être fragmentées sans contact par des ondes de choc, et pour positionner la concrétion (4) au foyer ($F_2$) d'un réflecteur d'ondes de choc (6) à deux systèmes de détection, caractérisé en ce qu'un système de détection fonctionne sur la base de rayons X et l'autre système de détection fonctionne sur la base d'ultra-sons à balayage-B, et est constitué de manière à pouvoir tourner, et en ce que les deux systèmes de détection sont mis en corrélation entre eux (20) et/ou avec un dispositif de positionnement.

2. Dispositif selon la revendication 1, caractérisé en ce que des points de référence de l'autre système sont respectivement mis au point sur les écrans (12, 16) des systèmes de détection (14, 8, 10).

3. Dispositif selon la revendication 2, caractérisé en ce que le foyer ($F_2$) est suivi automatiquement dans les deux écrans (12, 16) par des mouvements de rotation et en ce que le plan des ultra-sons ($U_1$, $U_2$) ou le rayon médian ($U_0$) du système à ultra-sons est mis au point sur l'écran à rayons X (12).

4. Dispositif selon l'une quelconque des revendications précédentes, caractérisé en ce que le rayon central (18) du système de détection à rayons X passe en totalité dans le plan image ($U_1$, $U_2$) du système à ultra-sons, de manière que le plan de l'image à rayons X et le plan de l'image à ultra-sons soient mutuellement perpendiculaires.

5. Dispositif selon l'une quelconque des revendications précédentes, caractérisé en ce que sont prévues des possibilités de rotation de la tête à ultra-sons (14) autour de son propre axe ($U_0$) et/ou autour du rayon central (18) du système de détection à rayons X et/ou autour de l'axe de rotation du réflecteur d'ondes de choc (6).

6. Dispositif selon la revendication 1, caractérisé en ce qu'il est prévu au moins un système de détection par ultra-sons et en ce que deux axes d'un dispositif de positionnement coïncident avec le plan de l'image par ultra-sons et les coordonnées du calcul rénal sur le plan de l'image par ultra-sons sont évaluées par un système de calcul et le calcul rénal est déplacé par un dispositif de régulation de manière qu'il parvienne au second foyer ($F_2$).

7. Dispositif selon l'une quelconque des revendications précédentes, caractérisé en ce qu'il est prévu au moins un système de détection par ultra-sons et un système de détection par rayons X, et en ce que deux axes du dispositif de positionnement passent par le plan de coupe des ultra-sons et le troisième axe du dispositif de positionnement passe le long du rayon central (18) du système de détection par rayons X, les coordonnées du calcul rénal étant déterminées par une unité de calcul et un mouvement de translation du calcul rénal dans le second foyer ($F_2$) étant obtenu par une commande.

8. Dispositif selon la revendication 1, caractérisé en ce qu'on utilise deux systèmes de détection par ultra-sons, dont les plans des images sont mutuellement perpendiculaires.

9. Dispositif selon l'une quelconque des revendications précédentes, caractérisé en ce que la surveillance de l'emplacement (4) du calcul est réalisée par une exploration automatique clarté/obscurité.

10. Dispositif selon l'une quelconque des revendications précédentes, caractérisé en ce que la concrétion (4) est amenée automatiquement au foyer ($F_2$) par un traitement d'image et des servomoteurs.

Fig.1

0 168 559

## Fig. 2

## Fig. 3

## Fig. 4